# EUROPÄISCHE PATENTANMELDUNG

(11) **EP 2 238 958 A2**
(43) Veröffentlichungstag der Anmeldung: **13.10.2010**
(21) Anmeldenummer: 10001623.7
(22) Anmeldetag: 17.02.2010
(51) Int. Cl.: A61F 17/00, B65D 83/14, B05B 11/00

(54) **Vorrichtung zur Aufnahme von Wechselbehältern und zur Ausgabe von Medien**

(30) Priorität: 17.07.2009 DE 102009033785; 06.07.2009 DE 102009031964; 09.04.2009 DE 102009016715
(71) Anmelder: Heidan, Michael, 65527 Niedernhausen-Engenhahn (DE); Okulla, Kai, 58791 Werdohl (DE)
(72) Erfinder: Heidan, Michael, 65527 Niedernhausen-Engenhahn (DE); Okulla, Kai, 58791 Werdohl (DE)
(74) Vertreter: Schmitz, Hans-Werner

(57) **Zusammenfassung**

Vorrichtung (1) zur Aufnahme von verschiedenen wechselbaren Behältern (4), welche mindestens zwei voneinander getrennte und verschließbare Innenräume (2a) aufweist, die auf einer gemeinsamen Längsachse (2b) angeordnet sind, ist offenbart.

Die Vorrichtung (1) ist **dadurch gekennzeichnet, dass** die Innenräume (2a) von jeweils einem Funktionsdeckel (3) verschlossen werden

## Beschreibung

Die Erfindung betrifft eine Vorrichtung zur Aufnahme von verschiedenen auswechselbaren Inhalten und/oder Behältern, gemäß dem Oberbegriff des Anspruches 1.

Es ist Aufgabe der vorliegenden Erfindung, eine Vorrichtung der im Oberbegriff des Anspruches 1 angegebenen Art zu schaffen, mit der es möglich ist, verschiedene wechselbare Inhalte und/oder Behälter, die unterschiedliche Medien enthalten, aufzunehmen und eine einhändige Bedienung zu ermöglichen.

Diese Aufgabe wird erfindungsgemäß durch die Merkmale des Patentanspruchs 1 gelöst. Vorteilhafte Ausführungsformen der Erfindung sind den Unteransprüchen zu entnehmen.

Die vorliegende Erfindung betrifft im Einzelnen eine Vorrichtung zur Aufnahme von verschiedenen auswechselbaren Inhalten und/oder Behältern und weist mindestens zwei getrennte und verschließbare Innenräume auf. Die Innenräume liegen auf einer gemeinsamen Längsachse und werden von Funktionsdeckeln verschlossen. Die Funktionsdeckel befinden sich an den Stirnseiten und weisen Öffnungen auf, durch die verschiedene Medien aus den Behältern austreten werden können. Die Öffnungen werden durch zusätzliche Abdeckungen verschlossen.

Die wechselbaren Behälter sind beispielsweise Sprayflaschen, welche Medien enthalten, die versprüht werden können.

Die genannten Sprayflaschen sind beispielsweise Desinfektionsmittel, Wund- und Säuberungsmittel und Sprühpflaster sowie Kältemittel und ermöglichen den Einsatz der Vorrichtung als Notfallset bei Verletzungen.

In Hinblick auf eine einfache Handhabung ist die Vorrichtung als einfaches Griffteil ausgeführt, welches eine eigenhändige Handhabung ermöglicht, wobei die Abdeckung beispielsweise an einer Lasche mit dem Daumen angehoben werden kann, um den Funktionsdeckel freizugeben. Der beispielsweise zweiteilige Funktionsdeckel ermöglicht eine Betätigung des Sprühpins der Sprayflasche, um das Medium zu versprühen.

Um die Vorrichtung vielfältig einsetzen zu können, sind verschiedene Adaptermöglichkeiten vorgesehen. So ist es möglich die Vorrichtung an einem Gürtel zu tragen oder an einem Fahrrad zu befestigen. Einsetzbar ist die Vorrichtung z.B. im Outdoorbereich, wie Wandern oder Radfahren. Hier können leichte Verletzungen auftreten, die den Einsatz von Desinfektionsmitteln und flüssigem Pflaster notwendig machen. Die Vorrichtung ist klein, leicht und einfach zu handhaben und daher geeignet für den mobilen Einsatz. Denkbar sind aber viele weitere Einsatzmöglichkeiten, mit verschiedenen Medien, wie z.B. im Tierbereich oder auch für Handwerker.

In einer besonders vorteilhaften Ausführung beinhaltet die Vorrichtung zur Aufnahme von verschiedenen wechselbaren Behältern, ein Griffteil, welches mindestens zwei voneinander getrennte und verschließbare Innenräume aufweist, die auf einer gemeinsamen Längsachse angeordnet sind. Die Innenräume werden von jeweils einem Funktionsdeckel verschlossen.

Vorteilhafterweise beinhaltet der zumindest eine Funktionsdeckel eine Öffnung, durch welche das in den Behältern befindliche Medium austreten kann.

Ein weiterer Vorteil besteht darin, dass der Funktionsdeckel einen Deckeleinsatz aufweist, welcher bei Betätigung den Sprühpin in eine beispielhafte Spraydose verschiebt, damit das Medium austreten und dosiert werden kann.

In einer besonderen Ausgestaltung ist der zumindest eine Funktionsdeckel mit einer Abdeckung verschließbar ausgeführt, damit die Öffnung z.B. vor Verschmutzung oder Zerstörung geschützt wird. Dabei ist der Funktionsdeckel, z.B. mit einem Filmscharnier, mit der Abdeckung beweglich verbunden.

In einer besonders vorteilhaften Ausgestaltung weist das Griffteil eine Adapteraufnahme auf, an welchem ein Adapter befestigt werden kann.

In einer weiteren besonders vorteilhaften Ausgestaltung weist der Adapter eine weitere Aufnahme auf.

Bei einer besonders bevorzugten Ausführungsform betrifft die vorliegende Erfindung ein Notfallset zur Aufnahme von verschiedenen auswechselbaren Inhalten. Es besteht aus einem Griffteil, welches drei voneinander getrennte und verschließbare Innenräume aufweist. Die Innenräume sind auf einer gemeinsamen Längsachse angeordnet. Der mittlere Innenraum kann durch einen Verschluss geöffnet und verschlossen werden. Dieser Verschluss kann beispielsweise als Schraubverschluss oder als Clipverschluss ausgeführt werden.

Die auswechselbaren Inhalte können unterschiedliche Kombinationen von Mitteln für die erste Hilfe bei Unfällen und Verletzungen sein. Beispielsweise können zwei Sprayflaschen mit Desinfektionsmittel und flüssigem Pflaster mit einer Warnweste oder einer Mullbinde kombiniert werden.

Eine weitere Möglichkeit besteht in der variablen Kombinationsmöglichkeit der Inhalte und der eingesetzten Behälter. So kann z. B. ein Notfallset und ein weiterer Tornister mittels eines Adapters bei einer Trinkflasche an Fahrrädern befestigt werden.

Die Einsatzgebiete des Notfallsets sind vielschichtig und werden hier nur beispielhaft genannt.

In einer besonders vorteilhaften Ausführung kann das Notfallset verschiedene auswechselbare Inhalte aufnehmen und besteht aus einem Griffteil, welches drei voneinander getrennte und verschließbare Innenräume aufweist, die auf einer gemeinsamen Längsachse angeordnet sind, wobei das Griffteil an dem mittleren Innenraum durch einen Verschluss geöffnet und verschlossen werden kann.

In einer besonders vorteilhaften Ausführung wird der Verschluss als Gewinde ausgeführt.

In einer weiteren besonders vorteilhaften Ausführung wird der Verschluss als Clip ausgeführt.

In einer besonderen Ausführung befindet sich in dem mittleren Innenraum eine Warnweste.

In einer weiteren besonderen Ausführung befindet sich in dem mittleren Innenraum eine Mullbinde.

In einer besonders bevorzugten Ausführung kann der Adapter sowohl ein Notfallset als auch zumindest einen weiteren Tornister aufnehmen.

In einer weiteren besonders bevorzugten Ausführung kann der Adapter sowohl ein Notfallset, einen Tornister und eine Trinkflasche aufnehmen.

Gemäß einer weiteren alternativen Ausführungsform betrifft die vorliegende Erfindung eine Vorrichtung zur Aufnahme von verschiedenen auswechselbaren Behältern und weist mindestens zwei getrennte und verschließbare Innenräume auf. Die Innenräume befinden sich in einer Art Aufnahmekörper, welcher mit der Trommel eines Revolvers verglichen werden kann. Dabei können die Innenräume auch doppelt angeordnet werden. Bei dieser Ausführung liegen je zwei Innenräume auf einer gemeinsamen Längsachse. Diese Ausführung wäre mir einer doppelten Trommel vergleichbar. Jeder Innenraum wird separat mit einem Funktionsdeckel verschlossen. Die Funktionsdeckel befinden sich an den Stirnseiten der Innenräume und weisen Öffnungen auf, durch die verschiedene Medien aus den Behältern austreten werden können. Die Öffnungen werden durch zusätzliche Abdeckungen verschlossen, um Verunreinigungen zu vermeiden.

Die wechselbaren Behälter sind beispielsweise Sprayflaschen, welche Medien enthalten, die versprüht werden können. Die Abdeckungen sind sogenannte Funktionsdeckel. Der beispielsweise zweiteilige Funktionsdeckel ermöglicht eine Betätigung des Sprühpins der Sprayflasche, um das Medium zu versprühen und verschließt gleichzeitig die Öffnung des Sprühpins.

Um die Vorrichtung vielfältig einsetzen zu können, sind verschiedene Ausstattungen vorgesehen. So ist es möglich die Vorrichtung, je nach Einsatzzweck, unterschiedlich zu befüllen. Ebenso kann durch eine geeignete Befestigungs- oder Tragemöglichkeit das Handling angepasst werden.

Die Notfallsetbox kann weiterhin derart erweitert werden, dass die Form und die Anordnung der Innenräume beliebig gestaltet werden kann. So ist auch eine doppelte Trommelform vorgesehen, bei der auf beiden Stirnseiten Innenräume mit Funktionsdeckeln angeordnet sind.

Einsetzbar ist die Notfallsetbox z. B. im Outdoorbereich, wie wandern oder Rad fahren. Hier können leichte Verletzungen auftreten, die den Einsatz von Desinfektionsmitteln und flüssigem Pflaster notwendig machen. Des Weiteren kann die Notfallsetbox auch beispielsweise eine Warnweste für Radfahrer aufnehmen. Viele andere Einsatzmöglichkeiten sind durch Nennung dieser Beispiele nicht ausgeschlossen. Die Vorrichtung ist klein, leicht und einfach zu handhaben und daher geeignet für den mobilen Einsatz. Denkbar sind aber viele weitere Einsatzmöglichkeiten, mit verschiedenen Medien, wie im Tierbereich oder auch für Handwerker.

Ein wesentlicher Vorteil besteht darin, dass die Notfallsetbox zur Aufnahme von verschiedenen wechselbaren Behältern, aus einem Grundkörper, welcher mindestens zwei voneinander getrennte und verschließbare Innenräume aufweist, die nebeneinander angeordnet sind und von jeweils einem Funktionsdeckel verschlossen werden.

In einer besonderen bevorzugten Ausgestaltung, weist der Funktionsdeckel zumindest eine Öffnung auf, durch welche das in den Behältern befindliche Medium austreten kann.

In einer weiteren Ausgestaltung weist der Funktionsdeckel eine Abdeckung auf.

In einer besonderen Ausführung kann der zumindest ein Innenraum Bestandteile einer Notfallausrüstung aufnehmen.

In einer weiteren besonders bevorzugten Ausführung sind die Innenräume des Grundkörpers um eine gemeinsame Achse angeordnet.

In einer weitereren vorteilhaften Ausgestaltung bildet der Grundkörper eine Dreiecksform.

In einer weitereren besonders vorteilhaften Ausgestaltung bildet der Grundkörper eine Kreisform.

In einer vorteilhaften Variante ist der Grundkörper doppelt ausgebildet, indem an dem Boden spiegelbildlich ein weiterer Grundkörper angeformt wird.

In der Zeichnung ist der Erfindungsgegenstand schematisch dargestellt und wird anhand der Figuren nachfolgend beschrieben, wobei gleich wirkende Elemente mit denselben Bezugszeichen versehen sind. Dabei zeigen:
- Fig. 1a: eine Gesamtdarstellung der erfindungsgemäßen Vorrichtung 1 mit der Position des Schnitts A-A,
- Fig. 1b: eine Gesamtdarstellung der erfindungsgemäßen Vorrichtung 1 im Schnitt A-A,
- Fig. 2: eine isometrische Explosionsdarstellung der erfindungsgemäßen Vorrichtung 1,
- Fig. 3a: eine isometrische Explosionsdarstellung eines erfindungsgemäßen Notfallsets,
- Fig. 3b: eine Schnittdarstellung des Notfallsets gemäß Fig. 3a,
- Fig. 4: eine Schnittdarstellung durch einen Tornister,
- Fig. 5: eine weitere isometrische Darstellung des erfindungsgemäßen Notfallsets,
- Fig. 6: eine isometrische Explosionsdarstellung einer weiteren Ausführungsform einer erfindungsgemäßen Vorrichtung bzw. Notfallsetbox,
- Fig. 7: einen Schnitt durch die erfindungsgemäße Notfallsetbox,
- Fig. 8: eine Dreiecksform des Grundkörpers der erfindungsgemäßen Notfallsetbox,
- Fig. 9: eine Kreisform des Grundkörpers der erfindungsgemäßen Notfallsetbox,
- Fig. 10: eine perspektivische Darstellung einer weiteren Ausführungsform der erfindungsgemäßen Vorrichtung bzw. der erfindungsgemäßen Notfallsetbox,
- Fig. 11: eine Schnittdarstellung der Ausführungsform gemäß Fig. 10,
- Fig. 12: das Detail B gemäß Fig. 11 in vergrößerter Darstellung,
- Fig. 13-15: den Fig. 10 bis 12 entsprechende Darstellungen einer weiteren Ausführungsform der erfindungsgemäßen Vorrichtung, und
- Fig. 16-18: den Fig. 10 bis 12 entsprechende Darstellungen einer weiteren alternativen Ausführungsform der erfindungsgemäßen Vorrichtung.

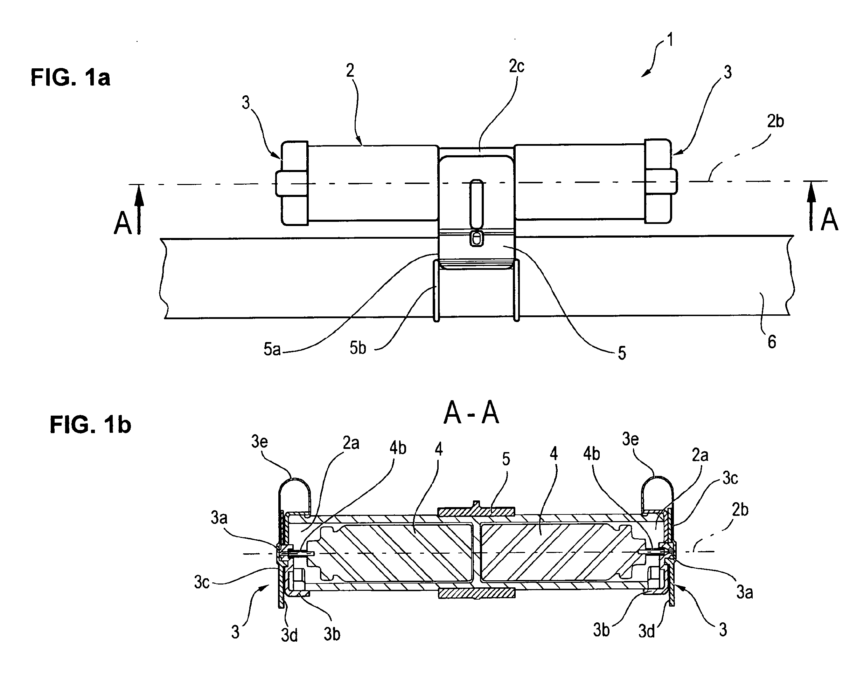

Fig. 1a zeigt eine Gesamtdarstellung der erfindungsgemäßen Vorrichtung 1 mit der Position des Schnittes A-A. Die Vorrichtung 1 ist mittels eines Adapters 5 an einem Rohr 6 befestigt. Das Rohr 6 ist z.B. Bestandteil eines Fahrradrahmens. Der Adapter 5 weist eine weitere Aufnahme 5a auf, die mit dem Zusatzteil 5b das Rohr 6 umschließt. Die andere Seite des Adapters 5 umgreift teilweise das Griffteil 2 an der Adapteraufnahme 2c. Die Vorrichtung 1 ist damit gegen axiales und radiales Verschieben gesichert. An beiden Enden der Vorrichtung 1 sind die Funktionsdeckel 3 dargestellt, die die Innenräume 2a, welche die Behälter 4 aufnehmen, verschließen.

Fig. 1b zeigt eine Gesamtdarstellung der erfindungsgemäßen Vorrichtung 1 im Schnitt A-A. In diesem Schnitt sind die Innenräume 2a dargstellt, welche die Behälter 4, mit den nicht dargestellten Medien 4a, aufnehmen. Die Funktionsdeckel 3 sind mit der Abdeckung 3c verschlossen. Das Griffteil 2 wird von dem Adapter 5 getragen.

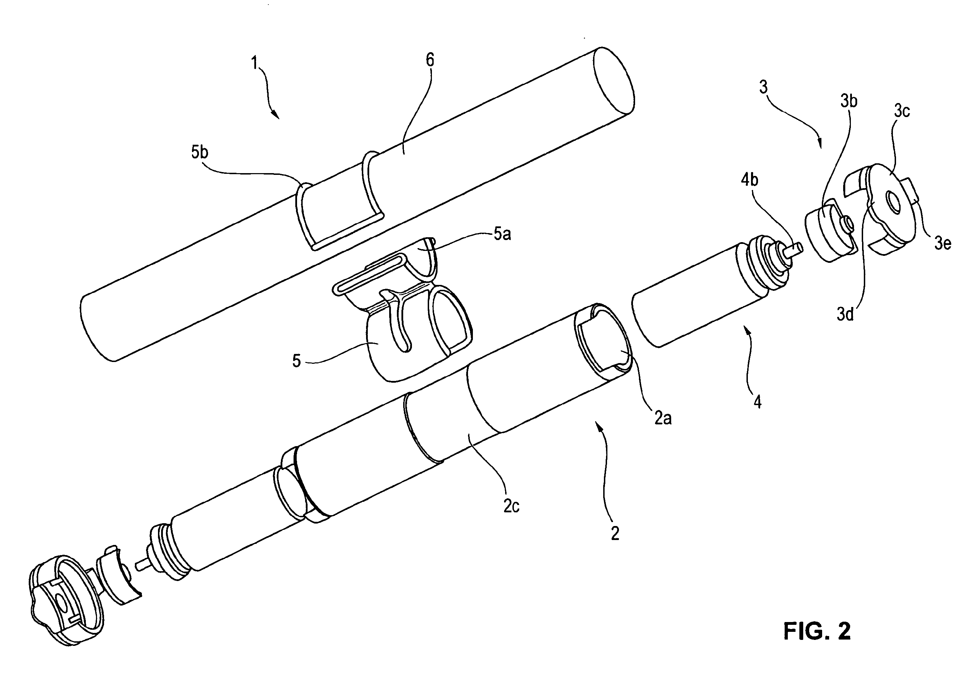

Fig. 2 zeigt eine isometrische Explosionsdarstellung der erfindungsgemäßen Vorrichtung 1. Hier sind alle Teile so dargestellt, dass der Zusammenbau und die Funktionen erkennbar sind. In die Innenräume 2a des Griffteils 2 werden die Behälter 4 eingelegt. Die Funktionsdeckel 3 verschließen die Innenräume 2a. Der Adapter 5 wird mit der weiteren Aufnahme 5a an das Rohr 6 positioniert und mit dem Zusatzteil 5b befestigt. Die Vorrichtung 1 wird dann an der Adapteraufnahme 2c in den Adapter 5 eingeführt.

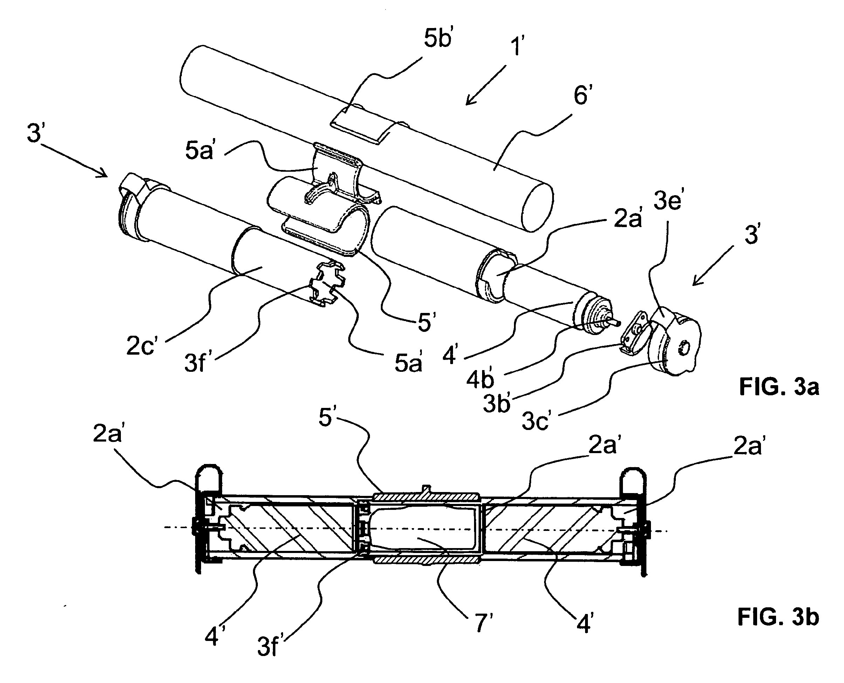

Fig. 3a zeigt eine isometrische Explosionsdarstellung des erfindungsgemäßen Notfallsets 1'. Das Notfallset 1' ist durch einen Adapter 5' an dem Rohr 6' eines Fahrrades befestigt. Das Griffteil 2' ist in Höhe des mittleren Innenraumes 2a' teilbar und wird durch einen Verschluss 3f geöffnet oder verschlossen. Die äußeren Innenräume nehmen Behälter 4', hier sind Sprühflaschen dargestellt, auf, welche durch die Funktionsdeckel 3' betätigt werden.

Fig. 3b zeigt eine Schnittdarstellung des erfindungsgemäßen Notfallsets 1'. In dieser Schnittdarstellung nimmt der mittlere Innenraum 2a' eine Warnweste 7' auf. In den beiden äußeren Innenräumen 2a' befinden sich Sprühflaschen.

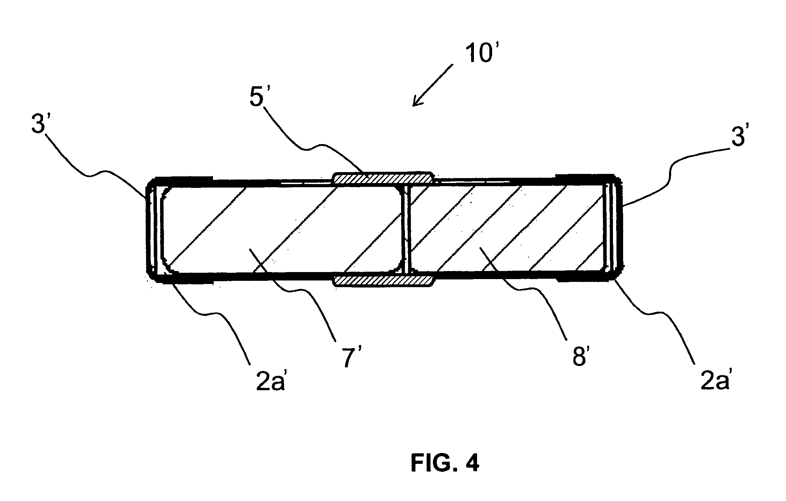

Fig. 4 zeigt eine Schnittdarstellung durch den Tornister 10'. Hier ist dargestellt, wie durch einen einfachen Deckel 3' die Innenräume 2a' verschlossen werden. Dabei befinden sich in den Innenräumen 2a' jeweils eine Warnweste 7' und eine Mullbinde 8'.

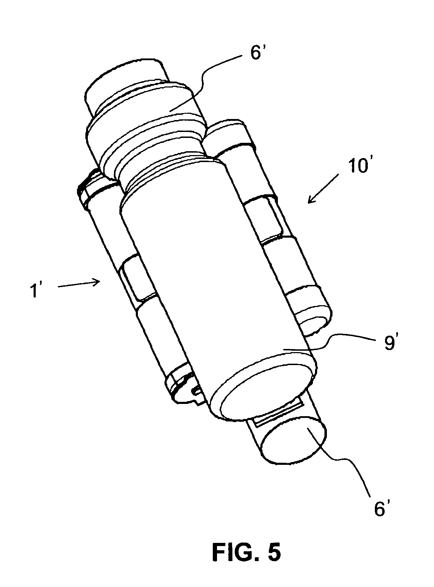

Fig. 5 zeigt eine isometrische Darstellung des erfindungsgemäßen Notfallsets 1'. In dieser Darstellung wird eine mögliche Anordnung an einem Fahrrad gezeigt. Der Adapter 5' ermöglicht hier die Aufnahme einer Trinkflasche 9', eines Notfallsets 1' und eines Tornisters 10'.

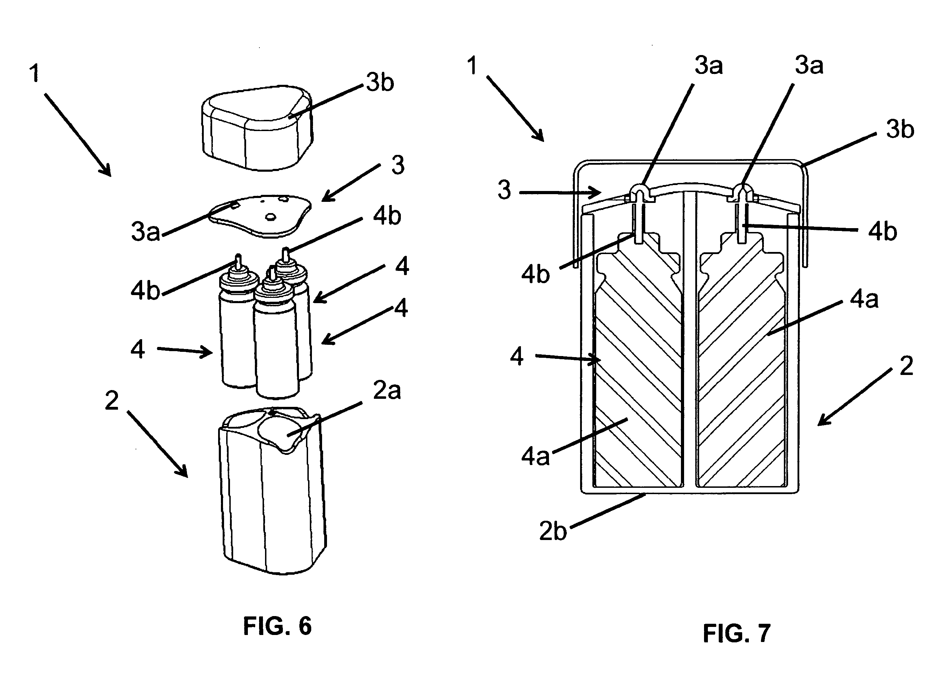

Fig. 6 zeigt eine isometrische Explosionsdarstellung einer erfindungsgemäßen Notfallsetbox 11. Die hier dargestellte Notfallsetbox 11 ist beinhaltet drei Innenräume 12a. Es sind drei Behälter 14 dargestellt, wie in diesen Innenräumen 12a aufgenommen werden können. Ein Funktionsdeckel 13 weist Öffnungen 13a auf, durch die das Medium 14a aus den Behältern 14 austreten kann. Die Abdeckung 13b dient als Schutz vor Verschmutzung und ist oberhalb der Öffnungen 13a abgebildet.

Fig. 7 zeigt einen Schnitt durch die erfindungsgemäße Notfallsetbox 11. Hier ist eine mögliche Anordnung abgebildet. In den Innenräumen 12a befinden sich die Behälter 14.

Die Sprühpins 14b sind unter dem Funktionsdeckel 13 angeordnet, so dass das Medium 14a durch die Öffnungen 13a, bei Betätigung des Funktionsdeckels 13, austreten kann. Die Abdeckung 13b verschließt die Notfallsetbox 11 und schützt vor Verschmutzung.

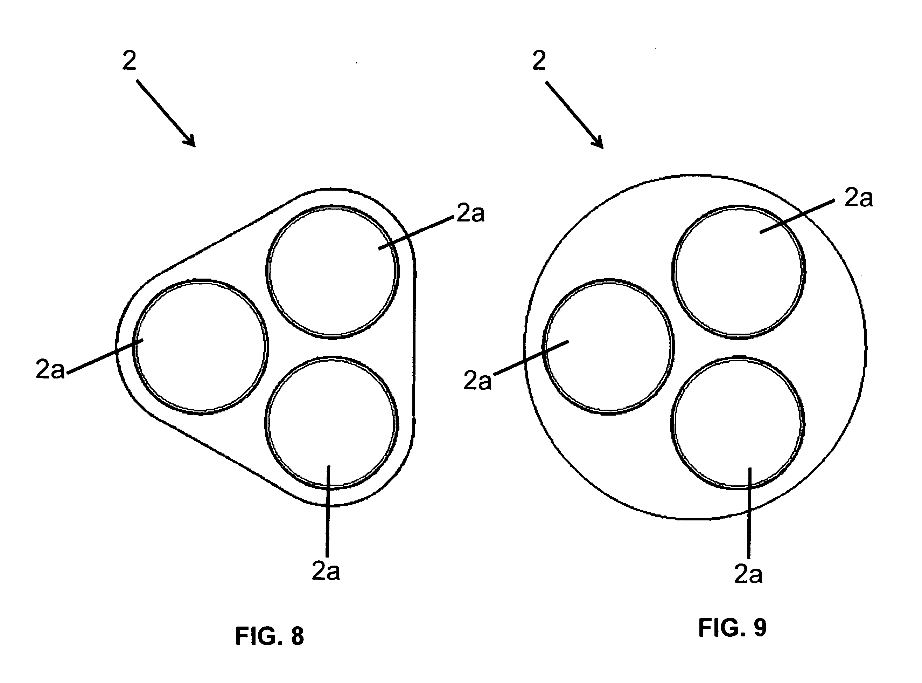

Fig. 8 zeigt eine Dreiecksform des Grundkörpers 12, der erfindungsgemäßen Notfallsetbox 11. In dieser Darstellung ist eine mögliche Form des Grundkörpers dargestellt.

Fig. 9 zeigt eine Kreisform des Grundkörpers 12, der erfindungsgemäßen Notfallsetbox 11. In dieser Darstellung ist eine weitere mögliche Form des Grundkörpers 12 dargestellt.

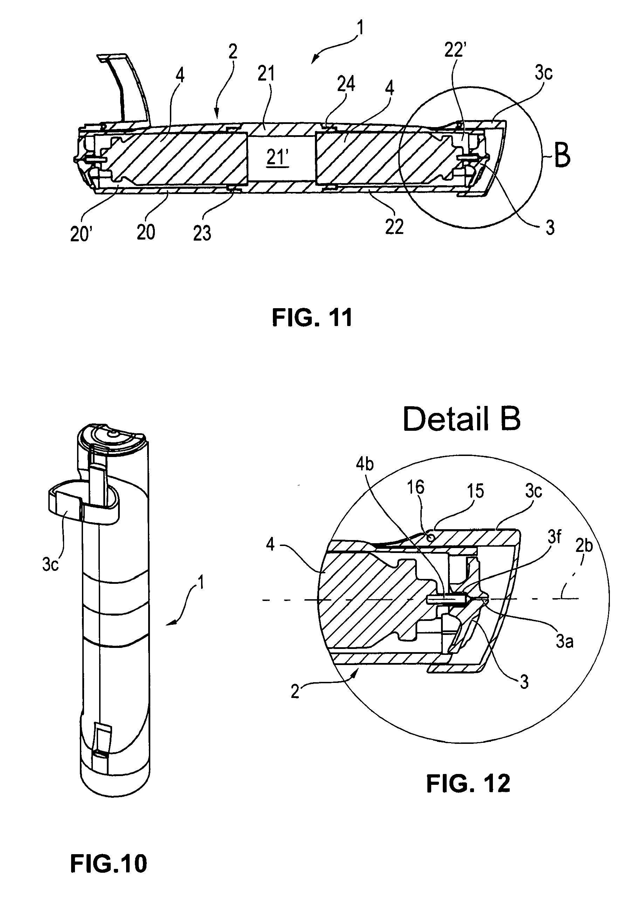

In Fig. 10 ist eine perspektivische Darstellung einer weiteren erfindungsgemäßen Vorrichtung 1 dargestellt, deren Abdeckung 3c im geöffneten Zustand dargestellt ist.

Fig. 11 zeigt eine Schnittdarstellung dieser Ausführungsform der Vorrichtung 1, bei der sämtliche mit den vorangehenden Ausführungsformen übereinstimmende Teile mit denselben Bezugsziffern versehen sind. Demgemäß weist die Vorrichtung 1 ein Griffteil bzw. ein Gehäuse 2 auf, das im Beispielsfalle drei Gehäuseteile 20, 21 und 22 mit entsprechenden Innenräumen 20', 21' und 22' aufweist. Die Gehäuseteile 20 und 21 sind hierbei über eine geeignete Verbindungseinrichtung 23, und die Gehäuseteile 21 und 22 über eine geeignete Verbindungseinrichtung 24 miteinander lösbar verbunden. Beispiele für derartige Verbindungseinrichtungen sind Schraubverbindungen, Steckverbindungen oder auch Bajonettverschlüsse.

In den beiden Innenräumen 20' und 22' sind jeweils Behälter für Sprühmittel, wie beispielsweise Sprühpflaster oder Kältemittel, angeordnet, die auch als Kartuschen bezeichnet werden können.

Der in Fig. 11 mit dem Kreis B umrandete Bereich des Gehäuses 2 ist in Fig. 12 in vergrößerter Darstellung wiedergegeben. Aus dieser Darstellung ergibt sich, dass der Funktionsdeckel 3 mit einer Öffnung 3a versehen ist, wobei der Funktionsdeckel 3 den Sprühpin 4b des Behälters 4 in einer mittigen Ausnehmung 3f aufnimmt. Wie Fig. 12 verdeutlicht, liegen der Sprühpin 4b mit seiner Öffnung und die Öffnung 3a auf der Längsachse 2b des Gehäuses 2, so dass sich bei Betätigen des Funktionsdeckels 3 eine Sprührichtung des im Behälter 4 befindlichen Mediums linear zur Längsachse 2b ergibt.

Ferner verdeutlicht die Darstellung der Fig. 12, dass der Deckel bzw. die Abdeckung 3c über eine Clipverbindung mit dem Gehäuse 2 schwenkbar verbunden ist. Diese Clipverbindung umfasst einen am Gehäuse 2 fixierten Steg 16 und eine am Deckel 3c angebrachte gabelförmige Stegaufnahme 15, die auf den Steg 16 aufgrund ihrer elastisch federnden Eigenschaften aufgeclipst werden kann.

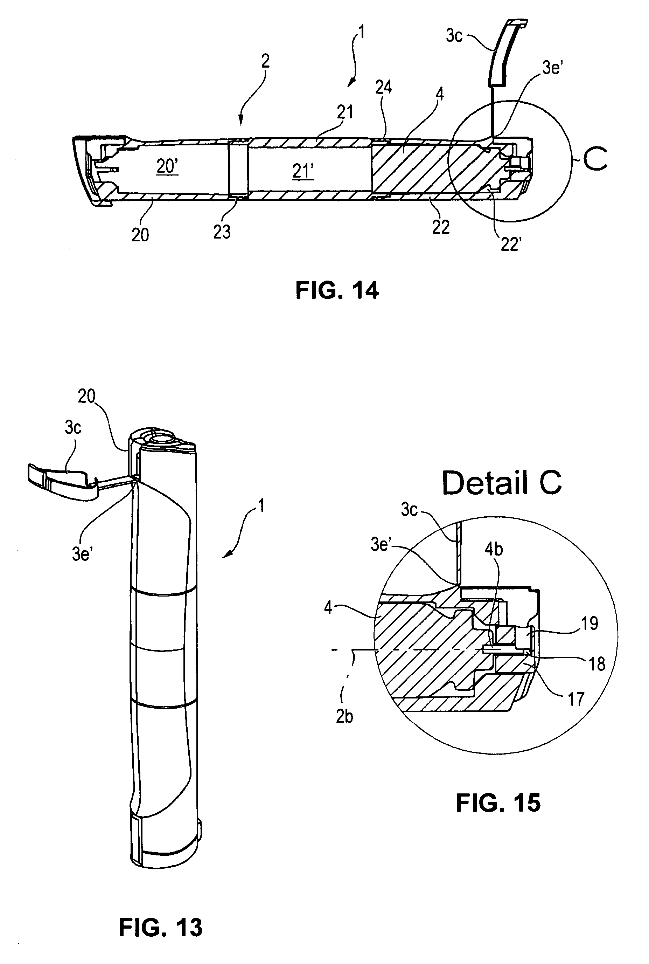

Die Fig. 13 zeigt eine der Fig. 10 entsprechende perspektivische Darstellung einer weiteren Ausführungsform der erfindungsgemäßen Vorrichtung, deren Abdeckung bzw. der Deckel 3c in diesem Beispiel mittels eines Filmscharniers 3e mit dem Gehäuse 2 verbunden ist.

Fig. 14 verdeutlicht eine der Fig. 11 entsprechende perspektivische Darstellung, wobei in diesem Falle lediglich eine Kartusche 4 im rechten Innenraum 22' angeordnet ist.

Fig. 15 stellt wiederum eine vergrößerte Darstellung des Details C aus Fig. 14 dar.

Aus dieser Darstellung wird deutlich, dass bei der Ausführungsform gemäß den Fig. 13 bis 15 eine Umlenkung der Sprührichtung um 90° zur Längsachse 2b möglich ist, wozu die Kartusche bzw. der Behälter 4 einen Deckel 17 aufweist, in dem zwei im rechten Winkel zueinander angeordnete Sprühmittelkanäle 18 und 19 vorgesehen sind. Der Sprühpin 4b greift in den Sprühmittelkanal 18 ein, so dass bei Betätigung des Deckels 17 der aus dem Behälter 4 austretende Flüssigkeitsstrahl durch die Kanäle 18 und 19 um 90° umgelenkt werden kann. Der Sprühstrahl tritt hierbei durch eine Aussparung 20 im Gehäuse 2 aus, die in Fig. 13 sichtbar ist.

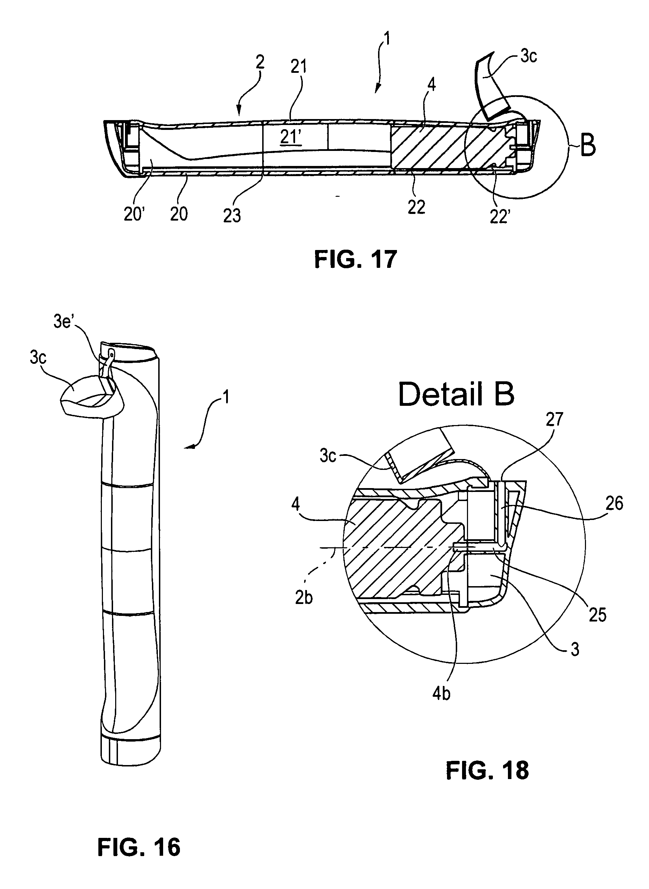

In den Fig. 16 bis 18 ist eine weitere Ausführungsform dargestellt, bei der wiederum sämtliche mit den vorangehenden Ausführungsformen übereinstimmende Teile mit denselben Bezugsziffern versehen sind.

Auch bei dieser Ausführungsform ist eine Umlenkung der Sprührichtung um 90° relativ zur Längsachse 2b vorgesehen. Bei dieser Ausführungsform sind hierfür im Funktionsdeckel 3 zwei im rechten Winkel zueinander angeordnete Sprühmittelkanäle 25 und 26 vorgesehen. Der Sprühpin 4b greift hierbei in den Sprühmittelkanal 25 ein. Wird dementsprechend der Funktionsdeckel 3 betätigt, beispielsweise durch Drücken auf seine Oberseite, tritt Sprühmittel über den Sprühpin 4b aus und wird über die im rechten Winkel zueinanderstehenden Kanäle 25 und 26 umgelenkt und tritt über die Öffnung 27 des Sprühmittelkanals 26 auf.

Die Erfindung wurde in Bezug auf eine besondere Ausführungsform beschrieben. Es ist jedoch selbstverständlich, dass Änderungen und Abwandlungen durchgeführt werden können, ohne dabei den Schutzbereich der nachstehenden Ansprüche zu verlassen. Hierbei ist hervorzuheben, dass sämtliche zuvor beschriebene Ausführungsformen und Einzelmerkmale beliebig miteinander kombinierbar sind.

### Bezugszeichenliste

- 1: Vorrichtung
- 2: Griffteil / Gehäuse
- 2a: Innenraum
- 2b: Längsachse
- 2c: Adapteraufnahme
- 3: Funktionsdeckel
- 3a: Öffnung
- 3b: Deckeleinsatz
- 3c: Abdeckung / Deckel
- 3d: Lasche
- 3e: Filmscharnier
- 3f: Ausnehmung
- 4: Behälter / Kartusche
- 4a: Medium
- 4b: Sprühpin
- 5: Adapter
- 5a: weitere Aufnahme
- 5b: Zusatzteil
- 6: Rohr
- 1': Notfallset
- 2': Griffteil
- 2a': Innenraum
- 2b': Längsachse
- 2c': Adapteraufnahme
- 3': Funktionsdeckel
- 3a': Öffnung
- 3b': Deckeleinsatz
- 3c': Abdeckung
- 3d': Lasche
- 3e': Filmscharnier
- 3f': Verschluss
- 4': Behälter
- 4a': Medium
- 4b': Sprühpin
- 5': Adapter
- 5a': weitere Aufnahme
- 5b': Zusatzteil
- 6': Rohr
- 7': Warnweste
- 8': Mullbinde
- 9': Trinkflasche
- 10': Tornister
- 11: Notfallsetbox
- 12: Grundkörper
- 12a: Innenraum
- 12b: Boden
- 13: Funktionsdeckel
- 13a: Öffnung
- 13b: Abdeckung
- 14: Behälter
- 14a: Medium
- 14b: Sprühpin
- 14c: Notfallausrüstung
- 15: gabelförmige Stegaufnahme
- 16: Steg
- 17: Deckel
- 18, 19: Sprühmittelkanäle / Kanäle
- 20, 21, 22: Gehäuseteil
- 20', 21', 22': Innenräume
- 23, 24: Verbindungseinrichtungen
- 25, 26: Sprühmittelkanäle / Kanäle

Neben der voranstehenden schriftlichen Offenbarung der Erfindung wird zu deren Ergänzung explizit auf die zeichnerische Darstellung in den Fig. 1 bis 18 Bezug genommen.

## Patentansprüche

1. Vorrichtung (1) zur Aufnahme von verschiedenen wechselbaren Inhalten und/oder Behältern, **gekennzeichnet durch** ein Griffteil (2), welches mindestens zwei voneinander getrennte und verschließbare Innenräume (2a) aufweist, und **dadurch gekennzeichnet, dass** die Innenräume (2a) von jeweils einem Funktionsdeckel verschließbar sind.

2. Vorrichtung (1) nach Anspruch 1, **dadurch gekennzeichnet, dass** der zumindest eine Funktionsdeckel (3) zumindest eine Öffnung (3a) aufweist, durch welche das in den Behältern (4) befindliche Medium (4a) austreten kann.

3. Vorrichtung (1) nach Anspruch 1 oder 2, **dadurch gekennzeichnet, dass** der Funktionsdeckel (3) einen Deckeleinsatz (3b) aufweist.

4. Vorrichtung (1) nach Anspruch 1, 2 oder 3, **dadurch gekennzeichnet, dass** der zumindest eine Funktionsdeckel (3) mit einer Abdeckung (3c) verschließbar ausgeführt ist.

5. Vorrichtung (1) nach den Ansprüchen 1 bis 4, **dadurch gekennzeichnet, dass** der Funktionsdeckel (3) mit der Abdeckung (3c) beweglich verbunden ist.

6. Vorrichtung (1) nach Anspruch 1, **dadurch gekennzeichnet, dass** das Griffteil (2) eine Adapteraufnahme (2c) aufweist, an welchem ein Adapter (5) befestigbar ist.

7. Vorrichtung (1) nach Anspruch 6, **dadurch gekennzeichnet, dass** der Adapter (5) eine weitere Aufnahme (5a) aufweist.

8. Vorrichtung nach Anspruch 1, **gekennzeichnet durch** drei voneinander getrennte und verschließbare Innenräume (2a'), die auf einer gemeinsamen Längsachse (2b') angeordnet sind, wobei das Griffteil (2') an den mittleren Innenraum (2a') **durch** einen Verschluss (3f') öffen- und schließbar ist.

9. Vorrichtung nach Anspruch 8, **dadurch gekennzeichnet, dass** der Verschluss als Gewinde oder Clip ausgebildet ist.

10. Vorrichtung nach einem der Ansprüche 8 oder 9, **dadurch gekennzeichnet, dass** sie als Notfallset ausgebildet ist und dass sich in dem mittleren Innenraum (2a') eine Warnweste (7') befindet.

11. Vorrichtung nach einem der Ansprüche 8 oder 9, **dadurch gekennzeichnet, dass** sie als Notfallset ausgebildet ist und dass sich in dem mittleren Innenraum (2a') eine Mullbinde (8') befindet.

12. Vorrichtung nach einem der Ansprüche 8 bis 11, **gekennzeichnet durch** einen Adapter (5') zur Aufnahme zumindest eines Notfallsets (1') und eines Tornisters (10').

13. Vorrichtung nach Anspruch 12, **dadurch gekennzeichnet, dass** der Adapter (5') ferner eine Trinkflasche aufnehmen kann.

14. Vorrichtung nach einem der Ansprüche 1 bis 13, **dadurch gekennzeichnet**, **dadurch gekennzeichnet, dass** die Innenräume (2a) auf einer gemeinsamen Längsachse (2b) angeordnet sind,

15. Vorrichtung nach einem der Ansprüche 1 bis 13, **dadurch gekennzeichnet, dass** die Innenräume (12a) nebeneinander angeordnet sind.
